# FASCICULE DE BREVET EUROPEEN

(11) **EP 1 030 646 B1**
(45) Date de publication et mention de la délivrance du brevet: **28.04.2004**
(21) Numéro de dépôt: 99923651.6
(22) Date de dépôt: 02.06.1999
(51) Int. Cl.: A61K 7/13

(54) **COMPOSITION DE TEINTURE D'OXYDATION DES FIBRES KERATINIQUES ET PROCEDE DE TEINTURE METTANT EN OEUVRE CETTE COMPOSITION**
OXIDATIONSFÄRBEMITTEL FÜR KERATINFASERN, UND VERFAHREN ZUR FÄRBUNG MIT DIESEM MITTEL
OXIDATION DYEING COMPOSITION FOR KERATINOUS FIBRES AND DYEING METHOD USING SAME

(30) Priorité: 19.06.1998 FR 9807795
(43) Date de publication de la demande: 30.08.2000
(73) Titulaire: L'OREAL, 75008 Paris (FR)
(72) Inventeur: AUDOUSSET, Marie-Pascale, F-92600 Asnières (FR)
(74) Mandataire: Goulard, Sophie
(86) Numéro de dépôt international: PCT/FR1999/001292
(87) Numéro de publication internationale: WO 1999/066892

(56) Documents cités:
- EP-A- 0 599 703
- EP-A- 0 634 164
- WO-A-97/35550
- WO-A-97/49378
- DE-A- 4 133 957

## Description

L'invention a pour objet une composition pour la teinture d'oxydation des fibres kératiniques, et en particulier des fibres kératiniques humaines telles que les cheveux comprenant, dans un milieu approprié pour la teinture, au moins une première base d'oxydation choisie parmi les pyrazolo-[1,5-a]-pyrimidines, du N,N'-bis-(β-hydroxyéthyl) N,N'-bis-(4'-aminophényl) 1,3-diamino propanol à titre de deuxième base d'oxydation, et au moins un coupleur choisi parmi les méta-aminophénols et les méta-phénylènediamines, ainsi que le procédé de teinture mettant en oeuvre cette composition.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des bases hétérocycliques, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Il a déjà été proposé, notamment dans la demande de brevet FR-A-2 750 048, des compositions pour la teinture d'oxydation des fibres kératiniques contenant, à titre de base d'oxydation, des pyrazolo-[1,5-a]-pyrimidines, éventuellement associées avec un ou plusieurs coupleurs. Cependant, les colorations obtenues ne sont pas toujours assez puissantes, chromatiques ou résistantes aux différentes agressions que peuvent subir les cheveux.

Or, la Demanderesse vient maintenant de découvrir qu'il est possible d'obtenir de nouvelles teintures, capables de conduire à des colorations puissantes, peu sélectives et résistant bien aux diverses agressions que peuvent subir les fibres, en associant au moins une pyrazolo-[1,5-a]-pyrimidine de formule (I) définie ci-après à titre de première base d'oxydation, du N,N'-bis-(β-hydroxyéthyl) N,N'-bis-(4'-aminophényl) 1,3-diamino propanol et/ou au moins un de ses sels d'addition avec un acide à titre de deuxième base d'oxydation, et au moins un coupleur choisi parmi des méta-aminophénols convenablement sélectionnés et les méta-phénylènediamines.

Cette découverte est à la base de la présente invention.

L'invention a donc pour premier objet une composition pour la teinture d'oxydation des fibres kératiniques et en particulier des fibres kératiniques humaines telles que les cheveux, caractérisée par le fait qu'elle comprend, dans un milieu approprié pour la teinture :
- au moins une première base d'oxydation choisie parmi les pyrazolo-[1,5-a]-pyrimidines de formule (I) suivante, leurs sels d'addition avec un acide ou avec une base : 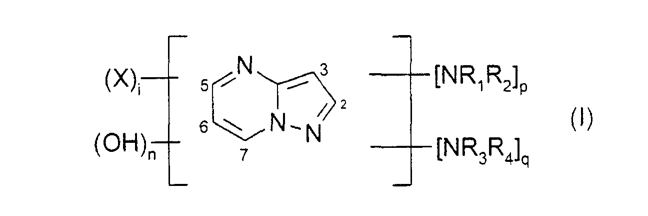 dans laquelle :
   - R₁, R₂ R₃ et R₄ désignent , identiques ou différents un atome d'hydrogène, un radical alkyle en C₁-C₄, un radical aryle, un radical hydroxyalkyle en C₁-C₄, un radical polyhydroxyalkyle en C₂-C₄, un radical (C₁-C₄)alcoxy alkyle en C₁-C₄, un radical amino alkyle en C₁-C₄ (l'amine pouvant être protégée par un acétyle, un uréido, un sulfonyl), un radical (C₁-C₄)alkyl amino alkyle en C₁-C₄, un radical di-[(C₁-C₄)alkyl] amino alkyle en C₁-C₄ (les dialkyles pouvant former un cycle aliphatique ou hétérocyclique à 5 ou 6 chaînons), un radical hydroxy(C₁-C₄)alkyl- ou di-[hydroxy(C₁-C₄) alkyl]-amino alkyle en C₁-C₄ ;
   - les radicaux X désignent , identiques ou différents, un atome d'hydrogène, un radical alkyle en C₁-C₄, un radical aryle, un radical hydroxyalkyle en C₁-C₄, un radical polyhydroxyalkyle en C₂-C₄, un radical amino alkyle en C₁-C₄, un radical (C₁-C₄)alkyl amino alkyle en C₁-C₄, un radical di-[(C₁-C₄)alkyl] amino alkyle en C₁-C₄ (les dialkyles pouvant former un cycle aliphatique ou hétérocyclique à 5 ou 6 chaînons), un radical hydroxy(C₁-C₄)alkyl ou di-[hydroxy(C₁-C₄)alkyl]amino alkyle en C₁-C₄, un radical amino, un radical (C₁-C₄)alkyl- ou di-[(C₁-C₄)alkyl]amino ; un atome d'halogène, un groupe acide carboxylique, un groupe acide sulfonique ;

   - i vaut 0, 1, 2 ou 3 ;
   - p vaut 0 ou 1 ;
   - q vaut 0 ou 1 ;
   - n vaut 0 ou 1 ;
   sous réserve que :
   - (i) la somme p + q est différente de 0 ;
   - (ii) lorsque p + q est égal à 2, alors n vaut 0 et les groupes NR₁R₂ et NR₃R₄ occupent les positions (2,3) ; (5,6) ; (6,7) ; (3,5) ou (3,7) ;
   - (iii) lorsque p + q est égal à 1 alors n vaut 1 et le groupe NR₁R₂ (ou NR₃R₄) et le groupe OH occupent les positions (2,3) ; (5,6) ; (6,7) ; (3,5) ou (3,7) ;
- au moins une deuxième base d'oxydation choisie parmi le N,N'-bis-(β-hydroxyéthyl) N,N'-bis-(4'-aminophényl) 1,3-diamino propanol et ses sels d'addition avec un acide ; et
- au moins un coupleur choisi parmi les méta-phénylènediamines et les méta-aminophénols de formule (II) suivante et leur sels d'addition avec un acide : 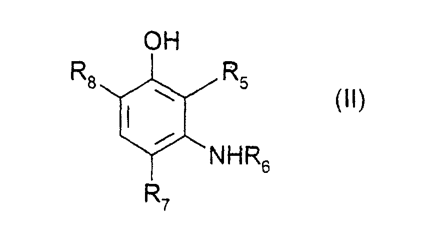 dans laquelle :
   - R₅ et R₈, identiques ou différents, représentent un atome d'hydrogène, un atome d'halogène tel que le chlore, le brome, l'iode ou le fluor, un radical alkyle en C₁-C₄, monohydroxyalkyle en C₁-C₄, polyhydroxyalkyle en C₂-C₄, alcoxy en C₁-C₄, monohydroxyalcoxy en C₁-C₄ ou polyhydroxyalcoxy en C₂-C₄ ;
   - R₆ représente un atome d'hydrogène, un radical alkyle en C₁-C₄, monohydroxyalkyle en C₁-C₄ ou polyhydroxyalkyle en C₂-C₄, aminoalkyle en C₁-C₄ ;
   - R₇ représente un atome d'hydrogène, un radical alkyle en C₁-C₄, alcoxy en C₁-C₄ ou un atome d'halogène choisi parmi le chlore, le brome ou le fluor ;
étant entendu que lorsque R₅ représente un atome de chlore et que R₆ et R₇ représentent simultanément un atome d'hydrogène, alors R₈ est différent d'un radical méthyle.

La composition tinctoriale conforme à l'invention conduit à des colorations puissantes, chromatiques, présentant une faible sélectivité et d'excellentes propriétés de résistances à la fois vis à vis des agents atmosphériques tels que la lumière et les intempéries et vis à vis de la transpiration et des différents traitements que peuvent subir les cheveux.

L'invention a également pour objet un procédé de teinture d'oxydation des fibres kératiniques mettant en oeuvre cette composition tinctoriale.

Les pyrazolo-[1,5-a]-pyrimidines de formule (I) utilisables à titre de première base d'oxydation dans la composition tinctoriale conforme à l'invention sont des composés connus et décrits dans la demande de brevet FR-A-2 750 048.

Parmi les pyrazolo-[1,5-a]-pyrimidines de formule (I), utilisables à titre de base d'oxydation dans les compositions tinctoriales conformes à l'invention, on peut notamment citer :
- la pyrazolo-[1,5-a]-pyrimidine-3,7-diamine ;
- la 2-méthyl pyrazolo-[1,5-a]-pyrimidine-3,7-diamine ;
- la 2,5-diméthyl pyrazolo-[1,5-a]-pyrimidine-3,7-diamine ;
- la pyrazoio-[1,5-a]-pyrimidine-3,5-diamine ;
- la 2,7-diméthyl pyrazolo-[1,5-a]-pyrimidine-3,5-diamine ;
- le 3-amino pyrazolo-[1,5-a]-pyrimidin-7-ol ;
- le 3-amino 5-méthyl pyrazolo-[1,5-a]-pyrimidin-7-ol ;
- le 3-amino pyrazolo-[1,5-a]-pyrimidin-5-ol ;
- le 2-(3-amino pyrazolo-[1,5-a]-pyrimidin-7-ylamino)-éthanol ;
- la 3-amino-7-β-hydroxyéthylamino-5-méthyl-pyrazolo-[1,5-a]-pyrimidine;
- le 2-(7-amino pyrazolo-[1,5-a]-pyrimidin-3-ylamino)-éthanol ;
- le 2-[(3-amino-pyrazolo-[1,5-a]-pyrimidin-7-yl)-(2-hydroxyéthyl)-amino]-éthanol ;
- le 2-[(7-amino-pyrazolo-[1,5-a]-pyrimidin-3-yl)-(2-hydroxyéthyl)-amino]-éthanol ;
- la 5,6-diméthyl pyrazolo-[1,5-a]-pyrimidine-3,7-diamine ;
- la 2,6-diméthyl pyrazolo-[1,5-a]-pyrimidine-3,7-diamine ;
- la 2, 5, N-7, N-7-tetraméthyl pyrazolo-[1,5-a]-pyrimidine-3,7-diamine ;
et leurs sels d'addition avec un acide ou avec une base.

Les méta-phénylènediamines utilisables à titre de coupleur dans la composition tinctoriale conforme à l'invention sont de préférence choisies parmi les composés de formule (III) suivante, et leurs sels d'addition avec un acide : 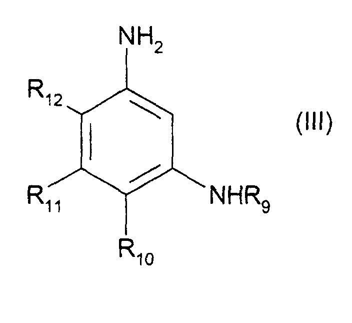 dans laquelle :
- R₉ représente un atome d'hydrogène, un radical alkyle en C₁-C₄, monohydroxyalkyle en C₁-C₄ ou polyhydroxyalkyle en C₂-C₄ ;
- R₁₀ et R₁₁, identiques ou différents, représentent un atome d'hydrogène, un radical alkyle en C₁-C₄, monohydroxyalcoxy en C₁-C₄ ou polyhydroxyalcoxy en C₂-C₄ ;
- R₁₂ représente un atome d'hydrogène, un radical alkoxy en C₁-C₄, aminoalkoxy en C₁-C₄, monohydroxyalkoxy en C₁-C₄, polyhydroxyalkoxy en C₂-C₄ ou un radical 2,4-diaminophénoxyalkoxy.

Parmi les méta-phénylènediamines de formule (III) ci-dessus, on peut plus particulièrement citer la métaphénylènediamine, le 3,5-diamino 1-éthyl 2-méthoxybenzène, le 3,5-diamino 2-méthoxy 1-méthyl benzène, le 2,4-diamino 1-éthoxybenzène, le 1,3-bis-(2,4-diaminophénoxy) propane, le bis-(2,4-diaminophénoxy) méthane, le 1-(β-aminoéthyloxy) 2,4-diamino benzène, le 2-amino 1-(β-hydroxyéthyloxy) 4-méthylamino benzène, le 2,4-diamino 1-éthoxy 5-méthyl benzène, le 2,4-diamino 5-(β-hydroxyéthyloxy) 1-méthylbenzène, le 2,4-diamino 1-(β,γ-dihydroxypropyloxy) benzène, le 2,4-diamino 1-(β-hydroxyéthyloxy) benzène, le 2-amino 4-N-(β-hydroxyéthyl) amino 1-méthoxy benzène, et leurs sels d'addition avec un acide.

Parmi les méta-aminophénols de formule (II) utilisables à titre de coupleur dans la composition tinctoriale conforme à l'invention, on peut plus particulièrement citer le méta-aminophénol, le 5-amino 2-méthoxy phénol, le 5-amino 2-(β-hydroxyéthyloxy) phénol, le 5-amino 2-méthyl phénol, le 5-N-(β-hydroxyéthyl)amino 2-méthyl phénol, le 5-N-(β-hydroxyéthyl)amino 4-méthoxy 2-méthyl phénol, le 5-amino 4-méthoxy 2-méthyl phénol, le 5-amino 4-chloro 2-méthyl phénol, le 5-amino 2,4-diméthoxy phénol, le 5-(γ-hydroxypropylamino) 2-méthyl phénol, 3-amino 6-chloro phénol, le 3-amino 6-bromo phénol, le 3-(β-aminoéthyl)amino 6-chloro phénol, le 3-(β-hydroxyéthyl)amino 6-chloro phénol et leurs sels d'addition avec un acide.

La ou les pyrazolo-[1,5-a]-pyrimidines de formule (I) conformes à l'invention et/ou le ou leurs sels d'addition avec un acide ou avec une base représentent de préférence de 0,0005 à 12 % en poids environ du poids total de la composition tinctoriale conforme à l'invention, et encore plus préférentiellement de 0,005 à 6 % en poids environ de ce poids.

Le N,N'-bis-(β-hydroxyéthyl) N,N'-bis-(4'-aminophényl) 1,3-diamino propanol et/ou le ou ses sels d'addition avec un acide utilisables à titre de deuxième base d'oxydation dans la composition tinctoriale conforme à l'invention représentent de préférence de 0,0005 à 12 % en poids environ du poids total de la composition tinctoriale conforme à l'invention, et encore plus préférentiellement de 0,005 à 6 % en poids environ de ce poids.

La ou les méta-phénylènediamines et/ou le ou les méta-aminophénols de formule (II) et/ou le ou leurs sels d'addition avec un acide représentent de préférence de 0,0001 à 10 % en poids environ du poids total de la composition tinctoriale et encore plus préférentiellement de 0,005 à 5 % en poids environ de ce poids.

La composition tinctoriale conforme à l'invention peut en outre contenir un ou plusieurs coupleurs différents des méta-phénylènediamines et des méta-aminophénols de formule (II) et/ou un ou plusieurs colorants directs, notamment pour modifier les nuances ou les enrichir en reflets.

Parmi les coupleurs pouvant être présents à titre additionnel dans la composition tinctoriale conforme à l'invention, on peut notamment citer les métadiphénols, les coupleurs hétérocycliques, et leurs sels d'addition avec un acide.

D'une manière générale, les sels d'addition avec un acide utilisables dans le cadre des compositions tinctoriales de l'invention (bases d'oxydation et coupleurs) sont notamment choisis parmi les chlorhydrates, les bromhydrates, les sulfates et les tartrates, les lactates et les acétates. Les sels d'addition avec une base utilisables dans le cadre des compositions tinctoriales de l'invention sont notamment ceux obtenus avec la soude, la potasse, l'ammoniaque ou les amines.

Le milieu approprié pour la teinture (ou support) de la composition tinctoriale conforme à l'invention est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ, et de préférence entre 5 et 12 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines, le 2-méthyl 2-amino propanol ainsi que leurs dérivés, les hydroxydes de sodium ou de potassium et les composés de formule (IV) suivante : 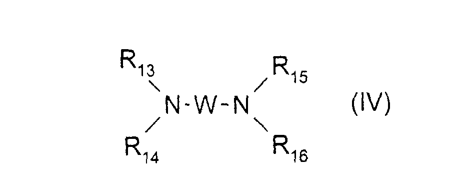 dans laquelle W est un reste propylène éventuellement substitué par un groupement hydroxyle ou un radical alkyle en C₁-C₄ ; R₁₃, R₁₄, R₁₅ et R₁₆, identiques ou différents, représentent un atome d'hydrogène, un radical alkyle en C₁-C₄ ou hydroxyalkyle en C₁-C₄.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques tels que par exemple des gommes de guar non-ioniques, des agents antioxydants, des enzymes telles que des oxydo-réductases à 2 électrons, des peroxydases ou des laccases, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Bien entendu, l'homme de l'art veillera à choisir ce ou ces éventuels composés complémentaires de manière telle que les propriétés avantageuses attachées intrinsèquement à la composition tinctoriale conforme à l'invention ne soient pas, ou substantiellement pas, altérées par la ou les adjonctions envisagées.

La composition tinctoriale conforme à l'invention peut se présenter sous des formes diverses, telles que sous forme de liquides, de poudres, de crèmes, de gels, éventuellement pressurisés, ou sous toute autre forme appropriée pour réaliser une teinture des fibres kératiniques, et notamment des cheveux humains.

L'invention a également pour objet un procédé de teinture des fibres kératiniques, et en particulier des fibres kératiniques humaines telles que les cheveux mettant en oeuvre la composition tinctoriale telle que définie précédemment.

Selon ce procédé, on applique sur les fibres la composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon une forme de mise en oeuvre particulièrement préférée du procédé de teinture selon l'invention, on mélange, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates, les peracides, les enzymes telles que les oxydo-réductases à 2 électrons, les peroxydases et les laccases. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

### EXEMPLES

### EXEMPLES 1 ET 2 DE TEINTURE

On a préparé les compositions tinctoriales suivantes (teneurs en grammes) :

| **EXEMPLE** | **1** | **2** |
|---|---|---|
| Pyrazolo-[1,5-a]-pyrimidine-3,7-diamine, 2HCl | 0,666 | 0,666 |
| N,N'-bis-(β-hydroxyéthyl) N,N'-bis-(4'-aminophényl) 1,3-diamino propanol | 0,759 | 0,759 |
| 2-méthyl 5-N-(β-hydroxyéthyl)amino phénol | 1,0 | - |
| 2,4-diamino 1-(β-hydroxyéthyloxy) benzène, 2HCl | - | 1,446 |
| Support de teinture commun | (*) | (*) |
| Eau déminéralisée q.s.p. | 100 g | 100 g |

| | | |
|---|---|---|
| (*) : Support de teinture commun : - Alcool éthylique à 96° 18 g - Sel pentasodique de l'acide diéthylène triamino pentacétique 1,1 g - Ammoniaque à 20% de NH₃ 10 g | | |

Au moment de l'emploi, on a mélangé poids pour poids chacune des compositions tinctoriales décrites ci-dessus avec une solution de peroxyde d'hydrogène à 20 volumes (6% en poids).

Chacun des mélange ainsi réalisé a été appliqué pendant 30 minutes sur des mèches de cheveux gris naturels à 90 % de blancs. Les mèches ont ensuite été rincées, lavées avec un shampooing standard, rincées à nouveau puis séchées.

Les cheveux ont été teints dans une nuance figurant dans le tableau ci-après :

| **EXEMPLE** | **NUANCE OBTENUE** |
|---|---|
| 1 | Irisé violacé |
| 2 | Bleu violacé |

## Revendications

1. Composition pour la teinture d'oxydation des fibres kératiniques et en particulier des fibres kératiniques humaines telles que les cheveux, **caractérisée par le fait qu'**elle comprend, dans un milieu approprié pour la teinture :
- au moins une première base d'oxydation choisie parmi les pyrazolo-[1,5-a]-pyrimidines de formule (I) suivante, leurs sels d'addition avec un acide ou avec une base : 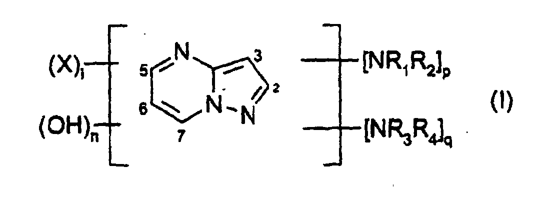 dans laquelle :
- R₁, R₂ R₃ et R₄ désignent , identiques ou différents un atome d'hydrogène, un radical alkyle en C₁-C₄, un radical aryle, un radical hydroxyalkyle en C₁-C₄, un radical polyhydroxyalkyle en C₂-C₄, un radical (C₁-C₄)alcoxy alkyle en C₁-C₄, un radical amino alkyle en C₁-C₄ (l'amine pouvant être protégée par un acétyle, un uréido, un sulfonyl), un radical (C₁-C₄)alkyl amino alkyle en C₁-C₄, un radical di-[(C₁-C₄)alkyl] amino alkyle en C₁-C₄ (les dialkyles pouvant former un cycle aliphatique ou hétérocyclique à 5 ou 6 chaînons), un radical hydroxy(C₁-C₄)alkyl- ou di-[hydroxy(C₁-C₄) alkyl]-amino alkyle en C₁-C₄ ;
- les radicaux X désignent , identiques ou différents, un atome d'hydrogène, un radical alkyle en C₁-C₄, un radical aryle, un radical hydroxyalkyle en C₁-C₄, un radical polyhydroxyalkyle en C₂-C₄, un radical amino alkyle en C₁-C₄, un radical (C₁-C₄)alkyl amino alkyle en C₁-C₄, un radical di-[(C₁-C₄)alkyl] amino alkyle en C₁-C₄ (les dialkyles pouvant former un cycle aliphatique ou hétérocyclique à 5 ou 6 chaînons), un radical hydroxy(C₁-C₄)alkyl ou di-[hydroxy(C₁-C₄)alkyl]amino alkyle en C₁-C₄, un radical amino, un radical (C₁-C₄)alkyl- ou di-[(C₁-C₄)alkyl]-amino ; un atome d'halogène, un groupe acide carboxylique, un groupe acide sulfonique ;
- i vaut 0, 1, 2 ou 3 ;
- p vaut 0 ou 1 ;
- q vaut 0 ou 1 ;
- n vaut 0 ou 1 ;
sous réserve que :
- (i) la somme p + q est différente de 0 ;
- (ii) lorsque p + q est égal à 2, alors n vaut 0 et les groupes NR₁R₂ et NR₃R₄ occupent les positions (2,3) ; (5,6) ; (6,7) ; (3,5) ou (3,7) ;
- (iii) lorsque p + q est égal à 1 alors n vaut 1 et le groupe NR₁R₂ (ou NR₃R₄) et le groupe OH occupent les positions (2,3) ; (5,6) ; (6,7) ; (3,5) ou (3,7) ;
- au moins une deuxième base d'oxydation choisie parmi N,N'-bis-(β-hydroxyéthyl) N,N'-bis-(4'-aminophényl) 1,3-diamino propanol et ses sels d'addition avec un acide ; et
- au moins un coupleur choisi parmi les méta-phénylènediamines et les méta-aminophénols de formule (II) suivante, et leur sels d'addition avec un acide : 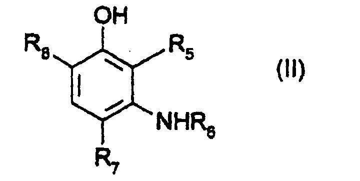 dans laquelle :
- R₅ et R₈, identiques ou différents, représentent un atome d'hydrogène, un atome d'halogène tel que le chlore, le brome, l'iode ou le fluor, un radical alkyle en C₁-C₄, monohydroxyalkyle en C₁-C₄, polyhydroxyalkyle en C₂-C₄, alcoxy en C₁-C₄, monohydroxyalcoxy en C₁-C₄ ou polyhydroxyalcoxy en C₂-C₄ ;
- R₆ représente un atome d'hydrogène, un radical alkyle en C₁-C₄, monohydroxyalkyle en C₁-C₄ ou polyhydroxyalkyle en C₂-C₄, aminoalkyle en C₁-C₄ ;
- R₇ représente un atome d'hydrogène, un radical alkyle en C₁-C₄, alcoxy en C₁-C₄ ou un atome d'halogène choisi parmi le chlore, le brome ou le fluor ;
étant entendu que lorsque R₅ représente un atome de chlore et que R₆ et R₇ représentent simultanément un atome d'hydrogène, alors R₈ est différent d'un radical méthyle.

2. Composition selon la revendication 1, **caractérisée par le fait que** les pyrazolo-[1,5-a]-pyrimidines de formule (I) sont choisies parmi :
- la pyrazolo-[1,5-a]-pyrimidine-3,7-diamine ;
- la 2-méthyl pyrazolo-[1,5-a]-pyrimidine-3,7-diamine ;
- la 2,5-diméthyl pyrazolo-[1,5-a]-pyrimidine-3,7-diamine ;
- la pyrazolo-[1,5-a]-pyrimidine-3,5-diamine ;
- la 2,7-diméthyl pyrazolo-[1,5-a]-pyrimidine-3,5-diamine ;
- le 3-amino pyrazolo-[1,5-a]-pyrimidin-7-ol ;
- le 3-amino 5-méthyl pyrazolo-[1,5-a]-pyrimidin-7-ol ;
- le 3-amino pyrazolo-[1,5-a]-pyrimidin-5-ol ;
- le 2-(3-amino pyrazolo-[1,5-a]-pyrimidin-7-ylamino)-éthanol ;
- la 3-amino-7-β-hydroxyéthylamino-5-méthyl-pyrazolo-[1,5-a]-pyrimidine;
- le 2-(7-amino pyrazolo-[1,5-a]-pyrimidin-3-ylamino)-éthanol ;
- le 2-[(3-amino-pyrazolo-[1,5-a]-pyrimidin-7-yl)-(2-hydroxyéthyl)-amino]-éthanol ;
- le 2-[(7-amino-pyrazolo-[1,5-a]-pyrimidin-3-yl)-(2-hydroxyéthyl)-amino]-éthanol ;.
- la 5,6-diméthyl pyrazolo-[1,5-a]-pyrimidine-3,7-diamine ;
- la 2,6-diméthyl pyrazolo-[1,5-a]-pyrimidine-3,7-diamine ;
- la 2, 5, N-7, N-7-tetraméthyl pyrazolo-[1,5-a]-pyrimidine-3,7-diamine ;
et leurs sels d'addition avec un acide ou avec une base.

3. Composition selon la revendication 1 ou 2, **caractérisée par le fait que** les méta-phénylènediamines sont choisies parmi les composés de formule (III) suivante, et leurs sels d'addition avec un acide : 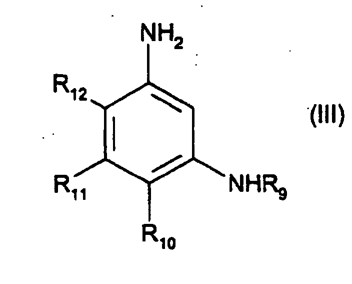 dans laquelle :
- R₉ représente un atome d'hydrogène, un radical alkyle en C₁-C₄, monohydroxyalkyle en C₁-C₄ ou polyhydroxyalkyle en C₂-C₄ ;
- R₁₀ et R₁₁, identiques ou différents, représentent un atome d'hydrogène, un radical alkyle en C₁-C₄, monohydroxyalcoxy en C₁-C₄ ou polyhydroxyalcoxy en C₂-C₄ ;
- R₁₂ représente un atome d'hydrogène, un radical alkoxy en C₁-C₄, aminoalkoxy en C₁-C₄, monohydroxyalkoxy en C₁-C₄, polyhydroxyalkoxy en C₂-C₄ ou un radical 2,4-diaminophénoxyalkoxy.

4. Composition selon la revendication 3, **caractérisée par le fait que** les méta-phénylènediamines de formule (III) sont choisies parmi la métaphénylènedlamine, le 3,5-diamino 1-éthyl 2-méthoxybenzène, le 3,5-diamino 2-méthoxy 1-méthyl benzène, le 2,4-diamino 1-éthoxybenzène, le 1,3-bis-(2,4-diaminophénoxy) propane, le bis-(2,4-diaminophénoxy) méthane, le 1-(β-aminoéthyloxy) 2,4-diainino benzène, le 2-amino 1-(β-hydroxyéthyloxy) 4-méthylamino benzène, le 2,4-diamino 1-éthoxy 5-méthyl benzène, le 2,4-diamino 5-(β-hydroxyéthyloxy) 1-méthylbenzène, le 2,4-diamino 1-(β,γ-dihydroxypropyloxy) benzène, le 2,4-diamino 1-(β-hydroxyéthyloxy) benzène, le 2-amino 4-N-(β-hydroxyéthyl) amino 1-méthoxy benzène, et leurs sels d'addition avec un acide.

5. Composition selon la revendication 1 ou 2, **caractérisée par le fait que** les méta-aminophénols de formule (II) sont choisis parmi le méta-aminophénol, le 5-amino 2-méthoxy phénol, le 5-amino 2-(β-hydroxyéthyloxy) phénol, le 5-amino 2-méthyl phénol, le 5-N-(β-hydroxyéthyl)amino 2-méthyl phénol, le 5-N-(β-hydroxyéthyl)amino 4-méthoxy 2-méthyl phénol, le 5-amino 4-méthoxy 2-méthyl phénol, le 5-amino 4-chloro 2-méthyl phénol, le 5-amino 2,4-diméthoxy phénol, le 5-(γ-hydroxypropylamino) 2-méthyl phénol, 3-amino 6-chloro phénol, le 3-amino 6-bromo phénol, le 3-(β-aminoéthyl)amino 6-chloro phénol, le 3-(β-hydroxyéthyl)amino 6-chloro phénol et leurs sels d'addition avec un acide.

6. Composition selon l'une quelconque des revendications précédentes, **caractérisée par le fait que** la ou les pyrazolo-[1,5-a]-pyrimidines de formule (I) et/ou le ou leurs sels d'addition avec un acide ou avec une base représentent de 0,0005 à 12 % en poids du poids total de la composition tinctoriale.

7. Composition selon la revendication 6, **caractérisée par le fait que** la ou les pyrazolo-[1,5-a]-pyrimidines de formule (I) et/ou le ou leurs sels d'addition avec un acide ou avec une base représentent de 0,005 à 6 % en poids du poids total de la composition tinctoriale.

8. Composition selon l'une quelconque des revendications précédentes, **caractérisée par le fait que** le N,N'-bis-(β-hydroxyéthyl) N,N'-bis-(4'-amino-phényl) 1,3-diamino propanol et/ou le ou ses sels d'addition avec un acide représentent de 0,0005 à 12 % en poids du poids total de la composition tinctoriale.

9. Composition selon la revendication 8, **caractérisée par le fait que** le N,N'-bis-(β-hydroxyéthyl) N,N'-bis-(4'-aminophényl) 1,3-diamino propanol et/ou le ou ses sels d'addition avec un acide représentent de 0,005 à 6 % en poids du poids total de la composition tinctoriale.

10. Composition selon l'une quelconque des revendications précédentes, **caractérisée par le fait que** la ou les méta-phénylènediamines et/ou le ou les méta-aminophénols de formule (II) et/ou le ou leurs sels d'addition avec un acide représentent de 0,0001 à 10 % en poids du poids total de la composition tinctoriale.

11. Composition selon la revendication 10, **caractérisée par le fait que** la ou les méta-phénytènediamines et/ou le ou les méta-aminophénols de formule (II) et/ou le ou leurs sels d'addition avec un acide représentent de 0,005 à 5 % en poids du poids total de la composition tinctoriale.

12. Composition selon l'une quelconque des revendications précédentes, **caractérisée par le fait que** les sels d'addition avec un acide sont choisis parmi les chlorhydrates, les bromhydrates, les sulfates et les tartrates, les lactates et les acétates et que les sels d'addition avec une base sont choisis parmi ceux obtenus avec la soude, la potasse. l'ammoniaque ou les amines.

13. Procédé de teinture des fibres kératiniques et en particulier des fibres kératiniques humaines telles que les cheveux, **caractérisé par le fait qu'**on applique sur lesdites fibres au moins une composition tinctoriale telle que définie à l'une quelconque des revendications 1 à 12, et que l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement.

14. Procédé selon la revendication 13, **caractérisé par le fait que** l'agent oxydant présent dans la composition oxydante est choisi parmi le peroxyde d'hydrogène, le peroxyde d'urée, les bromates de métaux alcalins, les persels, les peracides, et les enzymes.

15. Dispositif à plusieurs compartiments, ou "kit" de teinture à plusieurs compartiments, dont un premier compartiment renferme une composition tinctoriale telle que définie à l'une quelconque des revendications 1 à 12 et un second compartiment renferme une composition oxydante.

## Claims

1. Composition for the oxidation dyeing of keratinous fibres and in particular of human keratinous fibres, such as hair, **characterized in that** it comprises, in a medium appropriate for dyeing:
- at least one first oxidation base chosen from pyrazolo[1,5-a]pyrimidines of following formula (I) or their addition salts with an acid or with a base: 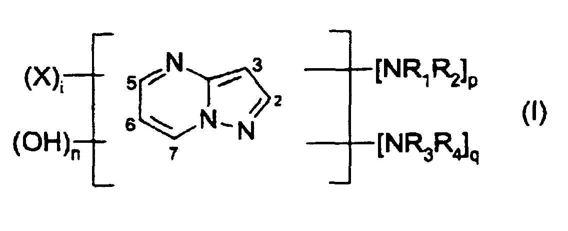 in which:
- R₁, R₂, R₃ and R₄, which are identical or different, denote a hydrogen atom, a (C₁-C₄)alkyl radical, an aryl radical, a hydroxy(C₁-C₄) alkyl radical, a polyhydroxy(C₂-C₄)alkyl radical, a (C₁-C₄)alkoxy(C₁-C₄)-alkyl radical, an amino(C₁-C₄)alkyl radical (it being possible for the amine to be protected by an acetyl, a ureido or a sulphonyl), a (C₁-C₄)alkylamino(C₁-C₄)alkyl radical, a di [(C₁-C₄)alkyl] amino (C₁-C₄) alkyl radical (it being possible for the dialkyls to form a 5- or 6-membered aliphatic or heterocyclic ring), a hydroxy(C₁-C₄)alkylamino(C₁-C₄)alkyl radical or a di[hydroxy(C₁-C₄)alkyl]amino(C₁-C₄)alkyl radical;
- the X radicals, which are identical or different, denote a hydrogen atom, a (C₁-C₄)alkyl radical, an aryl radical, a hydroxy(C₁-C₄)alkyl radical, a polyhydroxy(C₂-C₄) alkyl radical, an amino (C₁-C₄) alkyl radical, a (C₁-C₄)alkylamino(C₁-C₄)alkyl radical, a di[(C₁-C₄)alkyl]amino(C₁-C₄)alkyl radical (it being possible for the dialkyls to form a 5- or 6-membered aliphatic or heterocyclic ring), a hydroxy(C₁-C₄) alkylamino(C₁-C₄) alkyl radical, a di[hydroxy(C₁-C₄)alkyl]amino(C₁-C₄)alkyl radical, an amino radical, a (C₁-C₄)alkylamino radical, a di[(C₁-C₄)alkyl]amino radical, a halogen atom, a carboxylic acid group or a sulphonic acid group;
- i has the value 0, 1, 2 or 3;
- p has the value 0 or 1;
- q has the value 0 or 1;
- n has the value 0 or 1;
with the proviso that:
- (i) the sum p + q is other than 0;
- (ii) when p + q is equal to 2, then n has the value 0 and the NR₁R₂ and NR₃R₄ groups occupy the (2,3), (5,6), (6,7), (3,5) or (3,7) positions;
- (iii) when p + q is equal to 1, then n has the value 1 and the NR₁R₂ (or NR₃R₄) group and the OH group occupy the (2,3), (5,6), (6,7), (3,5) or (3,7) positions;
- at least one second oxidation base chosen from N,N'-bis(β-hydroxyethyl)-N,N'-bis(4'-aminophenyl)-1,3-diaminopropanol and its addition salts with an acid; and
- at least one coupler chosen from meta-phenylenediamines and meta-aminophenols of following formula (II) and their addition salts with an acid: in which: 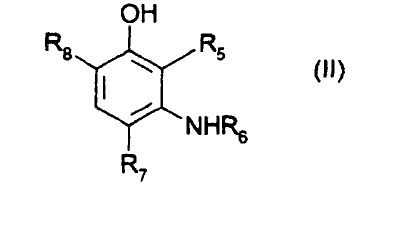
- R₅ and R₈, which are identical or different, represent a hydrogen atom, a halogen atom, such as chlorine, bromine, iodine or fluorine, or a (C₁-C₄)alkyl, monohydroxy(C₁-C₄)alkyl, polyhydroxy(C₂-C₄)alkyl, (C₁-C₄)alkoxy, monohydroxy(C₁-C₄)alkoxy or polyhydroxy(C₂-C₄)alkoxy radical;
- R₆ represents a hydrogen atom or a (C₁-C₄)alkyl, monohydroxy(C₁-C₄)alkyl, polyhydroxy(C₂-C₄)alkyl or amino(C₁-C₄)alkyl radical;
- R₇ represents a hydrogen atom, a (C₁-C₄)alkyl or (C₁-C₄)alkoxy radical or a halogen atom chosen from chlorine, bromine or fluorine;
it being understood that, when R₅ represents a chlorine atom and when R₆ and R₇ simultaneously represent a hydrogen atom, then R₈ is other than a methyl radical.

2. Composition according to Claim 1, **characterized in that** the pyrazolo[1,5-a]pyrimidines of formula (I) are chosen from:
- pyrazolo[1,5-a]pyrimidine-3,7-diamine;
- 2-methylpyrazolo[1,5-a]pyrimidine-3,7-diamine;
- 2,5-dimethylpyrazolo[1,5-a]pyrimidine-3,7-diamine;
- pyrazolo[1,5-a]pyrimidine-3,5-diamine;
- 2,7-dimethylpyrazolo[1,5-a]pyrimidine-3,5-diamine;
- 3-aminopyrazolo[1,5-a]pyrimidin-7-ol;
- 3-amino-5-methylpyrazolo[1,5-a]pyrimidin-7-ol;
- 3-aminopyrazolo[1,5-a]pyrimidin-5-ol;
- 2-(3-aminopyrazolo[1,5-a]pyrimidin-7-ylamino)ethanol;
- 3-amino-7-(β-hydroxyethylamino)-5-methylpyrazolo[1,5-a]pyrimidine;
- 2-(7-aminopyrazolo[1,5-a]pyrimidin-3-ylamino)ethanol;
- 2-[(3-aminopyrazolo[1,5-a]pyrimidin-7-yl)(2-hydroxyethyl)amino]ethanol;
- 2-[(7-aminopyrazolo[1,5-a]pyrimidin-3-yl)(2-hydroxyethyl)amino]ethanol;
- 5,6-dimethylpyrazolo[1,5-a]pyrimidine-3,7-diamine;
- 2,6-dimethylpyrazolo[1,5-a]pyrimidine-3,7-diamine;
- 2,5,N-7,N-7-tetramethylpyrazolo[1,5-a]pyrimidine-3,7-diamine;
and their addition salts with an acid or with a base.

3. Composition according to Claim 1 or 2, **characterized in that** the meta-phenylenediamines are chosen from the compounds of following formula (III) and their addition salts with an acid: 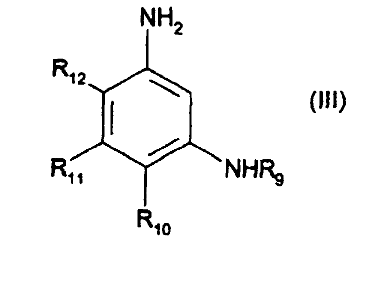 in which:
- R₉ represents a hydrogen atom or a (C₁-C₄)alkyl, monohydroxy(C₁-C₄) alkyl or polyhydroxy(C₂-C₄) alkyl radical;
- R₁₀ and R₁₁, which are identical or different, represent a hydrogen atom or a (C₁-C₄)alkyl, monohydroxy(C₁-C₄)alkoxy or polyhydroxy(C₂-C₄)alkoxy radical;
- R₁₂ represents a hydrogen atom, a (C₁-C₄)alkoxy, amino (C₁-C₄)alkoxy, monohydroxy(C₁-C₄)alkoxy or polyhydroxy(C₂-C₄)alkoxy radical or a 2,4-diamino-phenoxyalkoxy radical.

4. Composition according to Claim 3, **characterized in that** the meta-phenylenediamines of formula (III) are chosen from meta-phenylenediamine, 3,5-diamino-1-ethyl-2-methoxybenzene, 3,5-diamino-2-methoxy-1-methylbenzene, 2,4-diamino-1-ethoxybenzene, 1,3-bis(2,4-diaminophenoxy)propane, bis(2,4-diaminophenoxy)methane, 1-(β-aminoethyloxy)-2,4-diaminobenzene, 2-amino-1-(β-hydroxyethyloxy)-4-(methylamino)benzene, 2,4-diamino-1-ethoxy-5-methylbenzene, 2,4-diamino-5-(β-hydroxyethyloxy)-1-methylbenzene, 2,4-diamino-1-(β,γ-dihydroxypropyloxy)-benzene, 2,4-diamino-1-(β-hydroxyethyloxy)benzene, 2-amino-4-N-(β-hydroxyethyl)amino-1-methoxybenzene and their addition salts with an acid.

5. Composition according to Claim 1 or 2, **characterized in that** the meta-aminophenols of formula (II) are chosen from meta-aminophenol, 5-amino-2-methoxyphenol, 5-amino-2-(β-hydroxyethyloxy)phenol, 5-amino-2-methylphenol, 5-N-(β-hydroxyethyl)amino-2-methylphenol, 5-N-(β-hydroxyethyl)amino-4-methoxy-2-methylphenol, 5-amino-4-methoxy-2-methylphenol, 5-amino-4-chloro-2-methylphenol, 5-amino-2,4-dimethoxyphenol, 5-(γ-hydroxypropylamino)-2-methylphenol, 3-amino-6-chlorophenol, 3-amino-6-bromophenol, 3-(β-aminoethyl)amino-6-chlorophenol, 3-(β-hydroxyethyl)amino-6-chlorophenol and their addition salts with an acid.

6. Composition according to any one of the preceding claims, **characterized in that** the pyrazolo[1,5-a]pyrimidine or pyrazolo[1,5-a]pyrimidines of formula (I) and/or the addition salt or their addition salts with an acid or with a base represent from 0.0005 to 12% by weight of the total weight of the dyeing composition.

7. Composition according to Claim 6, **characterized in that** the pyrazolo[1,5-a]pyrimidine or pyrazolo[1,5-a]pyrimidines of formula (I) and/or the addition salt or their addition salts with an acid or with a base represent from 0.005 to 6% by weight of the total weight of the dyeing composition.

8. Composition according to any one of the preceding claims, **characterized in that** N,N'-bis(β-hydroxyethyl)-N,N'-bis(4'-aminophenyl)-1,3-diamino-propanol and/or the addition salt or its addition salts with an acid represent from 0.0005 to 12% by weight of the total weight of the dyeing composition.

9. Composition according to Claim 8, **characterized in that** N,N'-bis(β-hydroxyethyl)-N,N'-bis(4'-aminophenyl)-1,3-diaminopropanol and/or the addition salt or its addition salts with an acid represent from 0.005 to 6% by weight of the total weight of the dyeing composition.

10. Composition according to any one of the preceding claims, **characterized in that** the meta-phenylenediamine or meta-phenylenediamines and/or the meta-aminophenol or meta-aminophenols of formula (II) and/or the addition salt or their addition salts with an acid represent from 0.0001 to 10% by weight of the total weight of the dyeing composition.

11. Composition according to Claim 10, **characterized in that** the meta-phenylenediamine or meta-phenylenediamines and/or the meta-aminophenol or meta-aminophenols of formula (II) and/or the addition salt or their addition salts with an acid represent from 0.005 to 5% by weight of the total weight of the dyeing composition.

12. Composition according to any one of the preceding claims, **characterized in that** the addition salts with an acid are chosen from hydrochlorides, hydrobromides and sulphates and tartrates, lactates and acetates and **in that** the addition salts with a base are chosen from those obtained with sodium hydroxide, potassium hydroxide, aqueous ammonia or amines.

13. Process for dyeing keratinous fibres and in particular human keratinous fibres, such as hair, **characterized in that** at least one dyeing composition as defined in any one of Claims 1 to 12 is applied to the said fibres and **in that** the colour is developed at acidic, neutral or alkaline pH using an oxidizing agent which is added only at the time of use to the dyeing composition or which is present in an oxidizing composition applied simultaneously or sequentially in a separate fashion.

14. Process according to Claim 13, **characterized in that** the oxidizing agent present in the oxidizing composition is chosen from hydrogen peroxide, urea hydrogen peroxide, alkali metal bromates, persalts, peracids and enzymes.

15. Dyeing multi-compartment device or kit with several compartments, a first compartment of which includes a dyeing composition as defined in any one of Claims 1 to 12 and a second compartment of which includes an oxidizing composition.

## Patentansprüche

1. Zusammensetzung zum oxidativen Färben von Keratinfasern und insbesondere menschlichen Keratinfasern, wie dem Haar, **dadurch gekennzeichnet, dass** sie in einem zum Färben geeigneten Medium enthält:
- mindestens eine erste Oxidationsbase, die unter den Pyrazolo[1,5-a]-pyrimidinen der folgenden Formel (I) und den Additionssalzen dieser Verbindungen mit einer Säure oder mit einer Base ausgewählt ist: 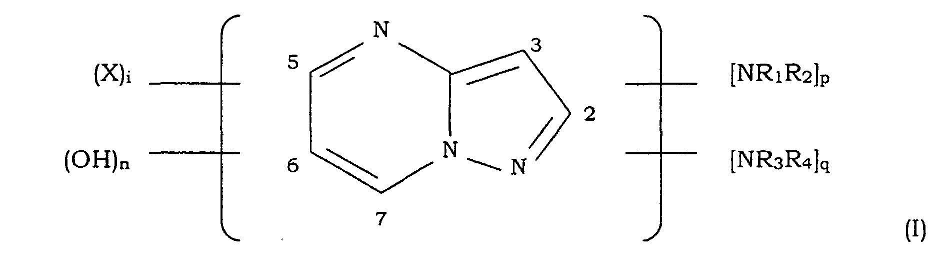 worin bedeuten:
- R₁, R₂, R₃ und R₄, die identisch oder voneinander verschieden sind, Wasserstoff, C₁₋₄-Alkyl, Aryl, C₁₋₄-Hydroxyalkyl, C₂₋₄-Polyhydroxyalkyl, C₁₋₄-Alkoxy-C₁₋₄-alkyl, C₁₋₄-Aminoalkyl (wobei die Aminogruppe mit Acetyl, Ureido oder Sulfonyl geschützt sein kann), C₁₋₄-Alkylamino-C₁₋₄-alkyl, Di-[C₁₋₄-alkyl]amino-C₁₋₄-alkyl (wobei die beiden Alkylgruppen einen aliphatischen oder heterocyclischen Ring mit 5 oder 6 Gliedern bilden können), Hydroxy-C₁₋₄-alkylamino-C₁₋₄-alkyl oder Di-[hydroxy-C₁₋₄-alkyl]amino-C₁₋₄-alkyl;
- die Gruppen X, die identisch oder voneinander verschieden sind, Wasserstoff, C₁₋₄-Alkyl, Aryl, C₁₋₄-Hydroxyalkyl, C₂₋₄-Polyhydroxyalkyl, C₁₋₄-Aminoalkyl, C₁₋₄-Alkylamino-C₁₋₄-alkyl, Di[C₁₋₄-alkyl]amino-C₁₋₄-alkyl (wobei die beiden Alkylgruppen einen aliphatischen oder heterocyclischen Ring mit 5 oder 6 Gliedern bilden können), Hydroxy-C₁₋₄-alkylamino-C₁₋₄-alkyl, Di-[hydroxy-C₁₋₄-alkyl]amino-C₁₋₄-alkyl, Amino, C₁₋₄-Alkylamino, Di-[C₁₋₄-alkyl]amino, Halogen, Carboxy oder eine Sulfonsäuregruppe;
- i 0, 1, 2 oder 3;
- p 0 oder 1:
- q 0 oder 1;
- n 0 oder 1;
mit der Maßgabe, dass
(i) die Summe p + q von Null verschieden ist;
(ii) n Null bedeutet und die Gruppen NR₁R₂ und NR₃R₄ die Stellungen (2,3); (5,6); (6,7); (3,5) oder (3,7) einnehmen, wenn p + q 2 ist;
(iii) n 1 bedeutet und die Gruppe NR₁R₂ (oder NR₃R₄) und die OH-Gruppe die Stellungen (2,3); (5,6); (6,7); (3,5) oder (3,7) einnehmen, wenn p + q 1 ist;
- mindestens eine zweite Oxidationsbase, die unter N,N'-Bis(β-hydroxyethyl)-N,N'-bis(4'-aminophenyl)-1,3-diamino-propanol und den Additionssalzen dieser Verbindung mit einer Säure ausgewählt ist; und
- mindestens einen Kuppler, der unter den m-Phenylendiaminen und den m-Aminophenolen der folgenden Formel (II) und deren Additionssalzen mit einer Säure ausgewählt ist: 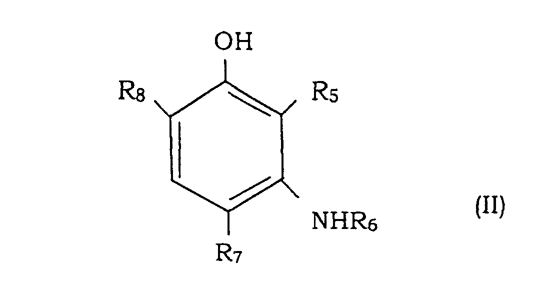
worin bedeuten:
- R₅ und R₈, die gleich oder verschieden sind, Wasserstoff, ein Halogenatom, wie Chlor, Brom, Iod oder Fluor, C₁₋₄-Alkyl, C₁₋₄-Monohydroxyalkyl, C₂₋₄-Polyhydroxyalkyl, C₁₋₄-Alkoxy, C₁₋₄-Monohydroxyalkoxy oder C₂₋₄-Polyhydroxyalkoxy;
- R₆ Wasserstoff, C₁₋₄-Alkyl, C₁₋₄-Monohydroxyalkyl, C₂₋₄-Polyhydroxyalkyl oder C₁₋₄-Aminoalkyl;
- R₇ Wasserstoff, C₁₋₄-Alkyl, C₁₋₄-Alkoxy oder ein Halogenatom, das unter Chlor, Brom oder Fluor ausgewählt ist;
mit der Maßgabe, dass R₈ nicht Methyl bedeutet, wenn R₅ ein Chloratom ist und R₆ und R₇ gleichzeitig Wasserstoff bedeuten.

2. Zusammensetzung nach Anspruch 1, **dadurch gekennzeichnet, dass** die Pyrazolo-[1,5-a]-pyrimidine der Formel (I) ausgewählt sind unter:
- Pyrazolo-[1,5-a]-pyrimidin-3,7-diamin,
- 2-Methyl-pyrazolo-[1,5-a]-pyrimidin-3,7-diamin,
- 2,5-Dimethyl-pyrazolo-[1,5-a]-pyrimidin-3,7-diamin,
- Pyrazolo-[1,5-a]-pyrimidin-3,5-diamin,
- 2,7-Dimethyl-pyrazolo-[1,5-a]-pyrimidin-3,5-diamin,
- 3-Amino-pyrazolo-[1,5-a]-pyrimidin-7-ol,
- 3-Amino-5-methyl-pyrazolo-[1,5-a]-pyrimidin-7-ol,
- 3-Amino-pyrazolo-[1,5-a]-pyrimidin-5-ol,
- 2-(3-Amino-pyrazolo-[1,5-a]-pyrimidin-7-ylamino)-ethanol,
- 3-Amino-7-β-hydroxyethylamino-5-methyl-pyrazolo-[1,5-a]-pyrimidin,
- 2-(7-Amino-pyrazolo-[1,5-a]-pyrimidin-3-ylamino)-ethanol,
- 2-[(3-Amino-pyrazolo-[1,5-a]-pyrimidin-7-yl)-(2-hydroxyethyl)amino]-ethanol,
- 2-[(7-Amino-pyrazolo-[1,5-a]-pyrimidin-3-yl)-(2-hydroxyethyl)amino]-ethanol,
- 5,6-Dimethyl-pyrazolo-[1,5-a]-pyrimidin-3,7-diamin,
- 2,6-Dimethyl-pyrazolo-[2,5-a]-pyrimidin-3,7-diamin,
- 2,5,N7,N7-Tetramethyl-pyrazolo-[1,5-a]-pyrimidin-3,7-diamin,
- und deren Additionssalzen mit einer Säure oder einer Base.

3. Zusammensetzung nach Anspruch 1 oder 2, **dadurch gekennzeichnet, dass** die m-Phenylendiamine unter den Verbindungen der Formel (III) und deren Additionssalzen mit einer Säure ausgewählt sind: 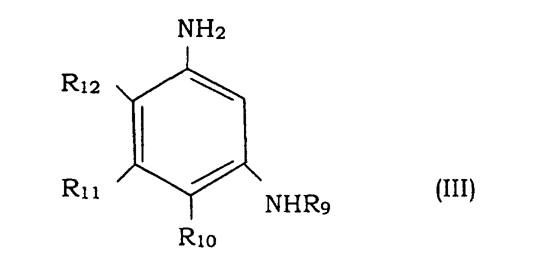 worin bedeuten:
- R₉ Wasserstoff, C₁₋₄-Alkyl, C₁₋₄-Monohydroxyalkyl oder C₂₋₄-Polyhydroxyalkyl,
- R₁₀ und R₁₁, die identisch oder voneinander verschieden sind, Wasserstoff, C₁₋₄-Alkyl, C₁₋₄-Monohydroxyalkoxy oder C₂₋₄-Polyhydroxyalkoxy,
- R₁₂ Wasserstoff, C₁₋₄-Alkoxy, C₁₋₄-Aminoalkoxy, C₁₋₄-Monohydroxyalkoxy, C₂₋₄-Polyhydroxyalkoxy oder 2,4-Diaminophenoxyalkoxy.

4. Zusammensetzung nach Anspruch 3, **dadurch gekennzeichnet, dass** die m-Phenylendiamine der Formel (III) ausgewählt sind unter: m-Phenylendiamin, 3,5-Diamino-1-ethyl-2-methoxy-benzol, 3,5-Diamino-2-methoxy-1-methyl-benzol, 2,4-Diamino-1-ethoxy-benzol, 1,3-Bis(2,4-diaminophenoxy)-propan, Bis(2,4-diaminophenoxy)-methan, 1-(β-Aminoethyloxy)-2,4-diamino-benzol, 2-Amino-1-(β-hydroxyethyloxy)-4-methylamino-benzol, 2,4-Diamino-1-ethoxy-5-methyl-benzol, 2,4-Diamino-5-(β-hydroxyethyloxy)-1-methyl-benzol, 2,4-Diamino-1-(β,γ-dihydroxy-propyloxy)-benzol, 2,4-Diamino-1-(β-hydroxyethyloxy)-benzol, 2-Amino-4-N-(β-hydroxyethyl)amino-1-methoxy-benzol und den Additionssalzen dieser Verbindungen mit einer Säure.

5. Zusammensetzung nach Anspruch 1 oder 2, **dadurch gekennzeichnet, dass** die m-Aminophenole der Formel (II) ausgewählt sind unter: m-Aminophenol, 5-Amino-2-methoxy-phenol, 5-Amino-2-(β-hydroxyethyloxy)-phenol, 5-Amino-2-methyl-phenol, 5-N-(β-Hydroxyethyl)amino-2-methyl-phenol, 5-N-(β-Hydroxyethyl)amino-4-methoxy-2-methyl-phenol, 5-Amino-4-methoxy-2-methyl-phenol, 5-Amino-4-chlor-2-methyl-phenol, 5-Amino-2,4-dimethoxy-phenol, 5-(γ-Hydroxypropylamino)-2-methyl-phenol, 3-Amino-6-chlorphenol, 3-Amino-6-brom-phenol, 3-(β-Aminoethyl)amino-6-chlorphenol, 3-(β-Hydroxyethyl)amino-6-chlor-phenol und den Additionssalzen dieser Verbindungen mit einer Säure.

6. Zusammensetzung nach einem der vorhergehenden Ansprüche, **dadurch gekennzeichnet, dass** das oder die Pyrazolo-[1,5-a]-pyrimidin(e) der Formel (I) und/oder das oder die Additionssalz(e) dieser Verbindung(en) mit einer Säure oder Base 0,0005 bis 12 Gew.-% des Gesamtgewichts der Farbmittelzusammensetzung ausmachen.

7. Zusammensetzung nach Anspruch 6, **dadurch gekennzeichnet, dass** das oder die Pyrazolo-[1,5-a]-pyrimidin(e) der Formel (I) und/oder das oder die Additionssalz(e) dieser Verbindung(en) mit einer Säure oder Base 0,005 bis 6 Gew.-% des Gesamtgewichts der Farbmittelzusammensetzung ausmachen.

8. Zusammensetzung nach einem der vorhergehenden Ansprüche, **dadurch gekennzeichnet, dass** das N,N'-Bis(β-hydroxyethyl)-N,N'bis(4'-aminophenyl)-1,3-diamino-propanol und/oder das oder die Additionssalz(e) dieser Verbindung mit einer Säure 0,0005 bis 12 Gew.-% des Gesamtgewichts der Farbmittelzusammensetzung ausmachen.

9. Zusammensetzung nach Anspruch 8, **dadurch gekennzeichnet, dass** das N,N'-Bis(β-hydroxyethyl)-N,N'-bis(4'-aminophenyl)-1,3-diamino-propanol und/oder das oder die Additionssalz(e) dieser Verbindung mit einer Säure 0,005 bis 6 Gew.-% des Gesamtgewichts der Farbmittelzusammensetzung ausmachen.

10. Zusammensetzung nach einem der vorhergehenden Ansprüche, **dadurch gekennzeichnet, dass** das oder die m-Phenylendiamine und/oder das oder die m-Aminophenole der Formel (II) und/oder das oder die Additionssalz(e) dieser Verbindung(en) mit einer Säure 0,0001 bis 10 Gew.-% des Gesamtgewichts der Farbmittelzusammensetzung ausmachen.

11. Zusammensetzung nach Anspruch 10, **dadurch gekennzeichnet, dass** das oder die m-Phenylendiamine und/ oder das oder die m-Aminophenole der Formel (II) und/oder das oder die Additionssalz(e) dieser Verbindung(en) mit einer Säure 0,005 bis 5 Gew.-% des Gesamtgewichts der Farbmittelzusammensetzung ausmachen.

12. Zusammensetzung nach einem der vorhergehenden Ansprüche, **dadurch gekennzeichnet, dass** die Additionssalze mit einer Säure unter den Hydrochloriden, Hydrobromiden, Sulfaten, Tartraten, Lactaten und Acetaten und die Additionssalze mit einer Base unter den Salzen ausgewählt sind, die mit Natriumhydroxid, Kaliumhydroxid, Ammoniak oder Aminen erhalten werden.

13. Verfahren zum oxidativen Färben von Keratinfasern und insbesondere menschlichen Keratinfasern, wie dem Haar, **dadurch gekennzeichnet, dass** auf die Fasern mindestens eine Farbmittelzusammensetzung nach einem der Ansprüche 1 bis 12 aufgebracht wird und die Farbe bei einem sauren, neutralen oder alkalischen pH-Wert mit einem Oxidationsmittel entwickelt wird, das bei der Anwendung zu der Farbmittelzusammensetzung gegeben wird oder das in einer oxidierenden Zusammensetzung vorliegt, die gleichzeitig oder getrennt davon anschließend aufgetragen wird.

14. Verfahren nach Anspruch 13, **dadurch gekennzeichnet, dass** das in der oxidierenden Zusammensetzung enthaltene Oxidationsmittel unter Wasserstoffperoxid, Harnstoffperoxid, Alkalimetallbromaten, Salzen von Persäuren, Persäuren und Enzymen ausgewählt ist.

15. Vorrichtung mit mehreren Abteilungen oder "Kit" zum Färben mit mehreren Abteilungen, wobei eine erste Abteilung eine Farbmittelzusammensetzung nach einem der Ansprüche 1 bis 12 und eine zweite Abteilung eine oxidierende Zusammensetzung enthält.
